## Europäisches Patentamt

## European Patent Office

## Office européen des brevets

(19)

(11) Veröffentlichungsnummer : **0 013 395
B1**

(12) **EUROPÄISCHE PATENTSCHRIFT**

(45) Veröffentlichungstag der Patentschrift :
**19.05.82**

(51) Int. Cl.³ : **C 07 C143/55**

(21) Anmeldenummer : **79105274.9**

(22) Anmeldetag : **19.12.79**

(54) **Verfahren zur Herstellung von 3-Nitro-naphthalin-1,5-disulfonsäure.**

(30) Priorität : **22.12.78 CH 13107/78**

(43) Veröffentlichungstag der Anmeldung :
**23.07.80 (Patentblatt 80/15)**

(45) Bekanntmachung des Hinweises auf die Patenterteilung : **19.05.82 Patentblatt 82/20**

(84) Benannte Vertragsstaaten :
**BE CH DE FR GB IT**

(56) Entgegenhaltungen :
**FR - A - 2 413 367
US - A - 1 756 537
US - A - 1 836 204
US - A - 2 191 820
US - A - 2 875 242**

**CHEMICAL ABSTRACTS, Band 60, Nr. 6, 16. März 1964, Zusammenfassung Nr. 6802e-f, Columbus, Ohio, US**

(73) Patentinhaber : **CIBA-GEIGY AG
Patentabteilung Postfach
CH-4002 Basel (CH)**

**BAYER AG
Zentralbereich Patente, Marken und Lizenzen
D-5090 Leverkusen 1, Bayerwerk (DE)**

(72) Erfinder : **Albrecht, Bernhard
Im Laig 173
CH-4463 Buus (CH)**
Erfinder : **Frey, Hans
Hauptstrasse 18
CH-4126 Bettingen (CH)**
Erfinder : **Habermacher, Vinzenz
Hammerstrasse 42
CH-4058 Basel (CH)**
Erfinder : **Behre, Horst, Dr.
Im Hellsiefen 4
5068 Odenthal (DE)**
Erfinder : **Kienitz, Lutz, Dr.
Forststrasse 2
5060 Bergisch Gladbach 1 (DE)**
Erfinder : **Schenk, Wolfgang, Dr.
Odenthaler Strasse 62/64
5090 Leverkusen (DE)**
Erfinder : **Steffan, Guido, Dr.
Im Herzogenfeld 52
5068 Odenthal (DE)**
Erfinder : **Vogel, Axel, Dr.
In der Hildscheid 5
5068 Odenthal (DE)**

(74) Vertreter : **Zumstein sen., Fritz, Dr. et al
Bräuhausstrasse 4
D-8000 München 2/(DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 013 395 B1

## Verfahren zur Herstellung von 3-Nitro-naphthalin-1,5-disulfonsäure

Nitro-Armstrongsäure (3-Nitro-naphthalin-1,5-disulfonsäure) stellt eine Zwischenstufe zur Herstellung der entsprechenden 3-Aminonaphthalin-1,5-disulfonsäure der sogenannten C-Säure dar.

C-Säure wird z.B. als Diazotierungskomponente für Baumwoll-Farbstoffe oder als Zwischenprodukt zur Herstellung von 2-Naphthol-4,8-disulfonsäure verwendet. Sie wird grosstechnisch nach einem in Ullmanns Encyklopädie der technischen Chemie, 3. Auflage, Band 12, Seite 629, beschriebenen Verfahren hergestellt, indem man Naphthalin in einem Ueberschuss an 20 % igem Oleum bei 20 bis 50 °C zu Naphthalin-1,5-disulfonsäure sulfoniert, diese zu 3-Nitronaphthalin-1,5-disulfonsäure nitriert und anschliessend reduziert. Dieses Verfahren ergibt eine wenig befriedigende Gesamtausbeute.

Die bekannte Sulfonierung von Naphthalin ergibt das sogenannte Armstrongsäureanhydrid (im weiteren Verlauf auch als AS-AN bezeichnet), welches 75 bis 80 % 1,5-Naphthalindisulfonsäure in der Form eines Anhydrids mit 1 Mol $H_2SO_4$ enthält. Die Aufarbeitung zur Trockensubstanz und deren Lagerung und Dosierung erweist sich als sehr aufwendig. Die Handhabung wie auch das Filtrieren, Trocknen, Lagerung in Silos und Dosieren des AS-AN erfordern kostspielige Spezialapparate, da es stark hygroskopisch ist und ausserdem z.B. bei mechanischer Beanspruchung und Temperaturen oberhalb 30 °C, leicht zu einer gummiartigen, praktisch nicht mehr weiterverarbeitbaren Masse führt. Die notwendige Niedertemperaturtrocknung benötigt zudem viel Energie.

Zur Umgehung dieser Feststoffhandhabung wäre es naheliegend für die nachfolgende Nitrierung direkt das Sulfonierungsgemisch zu verwenden. Dies scheitert jedoch, weil mit zu geringer Schwefelsäuremenge eine gummiartige Masse resultiert; die Verwendung geeigneter Schwefelsäuremengen von mehr als 15 Mol/Mol AS-AN ist grosstechnisch aber unwirtschaftlich und ökologisch nicht mehr tragbar.

Es wurde nun gefunden, dass durch Rückführung eines Teils der rohen Nietriermasse eines vorausgegangenen Nitrieransatzes überraschenderweise die erforderliche Schwefelsäuremenge drastisch reduziert werden kann, und dass AS-AN zudem in einem für die Weiterverarbeitung gut geeigneten pumpbaren Brei erhalten wird.

Das neue Verfahren zur Herstellung von Nitro-Armstrongsäure ist dadurch gekennzeichnet, dass man Naphthalin in einem reaktionsinerten organischen Lösungsmittel mit flüssigem Schefeltrioxid sulfoniert, wobei ein Molverhältnis von $SO_3$ zu Naphthalin im Bereich von 2,5 bis 3,6, vorzugsweise im Bereich von 2,5 bis 3,3, eingehalten wird. Dabei bildet sich ein Sulfonierungsprodukt des Naphthalins, welches sich in fester Form abscheidet und $H_2SO_4$ und gegebenenfalls $SO_3$ enthält. Das Sulfonierungsprodukt liegt als Suspension in dem verwendeten Lösungsmittel vor. Dem erhaltenen Reaktionsgemisch wird eine Mischung, bestehend aus in etwa gleichen Teilen 100%iger Schwefelsäure und ausreagierter roher Nitriermasse aus der Nitrierung einer vorausgegangenen Nitrierungscharge zugesetzt, wodurch ein Zweiphasen-System resultiert, von dem die obere organische Lösungsmittel-Phase dekantiert und der Rest des verbliebenen organischen Lösungsmittels aus dem AS-AN Säurebrei destillativ entfernt wird, vorteilhaft durch Flashverdampfung oder gewünschtenfalls bei der nachfolgenden Nitrierung zur gleichzeitigen Abführung der Nitrierungswärme, wonach man zum Rückstand die zur Nitrierung erforderliche Salpetersäuremenge zweckmässig als Mischsäure bei einer Temperatur zwischen 10 und 60 °C zusetzt und die entstandene 3-Nitro-naphthalin-1,5-disulfonsäure durch Ausfällung, vorteilhaft als Magnesiumsalz, bei 90 bis 100 °C und Filtration isoliert.

Das zur Phasentrennung nach der Sulfonierungsreaktion einzusetzende Säuregemisch aus konzentrierter Schwefelsäure und ausreagierter Nitriermasse lässt sich variieren, doch soll die Gesamtmenge des Gemisches vorteilhaft aus mindestens 6 Gewichtsteilen, und die beiden einzelnen Komponenten aus mindestens je 3 Gewichtsteilen pro 1 Teil in die Sulfonierungsreaktion eingesetztem Naphthalin bestehen. Werden die beiden Komponenten nicht als Säuregemisch sondern als Einzelkomponenten in beliebiger Reihenfolge der Sulfonierungsmasse zugesetzt, dann entsteht eine unrührbare Masse. Die Verwendung des Säuregemisches ist somit von entscheidender Bedeutung.

Zur Nitrierung verwendet man vorteilhaft 1 bis 1,5 Mol Salpetersäure conc. in Form von Mischsäure pro Mol eingesetztem Naphthalin. Zweckmässig soll die Mischsäure 0,8 bis 1,2 Mol $SO_3$ pro Mol Salpetersäure enthalten.

Eine bevorzugte Ausführungsform des neuen Verfahrens besteht darin, dass man den nach der Phasentrennung erhaltenen AS-AN-Brei und die Mischsäure entweder taktweise hintereinander oder simultan in ein vorgelegtes rohes Nitriergemisch dosiert und anschliessend wie bekannt nitriert.

Bei dieser Verfahrensvariante wird das rohe Nitriergemisch in 3 Teile aufgeteilt. Ein Teil wird zum Produkt 3-Nitronaphthalin-1,5-disulfonsäure aufgearbeitet, indem man es in Wasser bei 80 bis 100 °C löst und als Magnesiumsalz isoliert. Der zweite Teil wird mit 100 %iger Schwefelsäure im Verhältnis 1 : 1 gemischt und zum Zwecke der Phasentrennung dem Sulfonierungsgemisch zugegeben. Der dritte Teil dient als Vorlage für die genannte bevorzugte Ausführungsform einer nächsten Nitrierungsreaktion.

Die vorher genannte Entfernung der letzten Reste des Lösungsmittels aus dem Reaktionssystem, die nach der Dekantierung noch verbleiben, stellt kein grundsätzliches Problem dar. Deshalb kann die Entfernung dieser Reste fakultativ entweder vor der Nitrierungsreaktion durch eine sogenannte Flashverdampfung (Schnellverdampfung) unter Anwendung von Vakuum oder aber erst während der Nitrierung durch Destillation unter Ausnützung der Nitrierungswärme erfolgen, wodurch externe Kühlsysteme zur Abkühlung der Nitrierungsreaktions eingespart werden können.

Die Trennoperation des Zweiphasen-flüssig-Gemisches nach der Sulfonierungsreaktion erfolgt bei Raumtemperatur (ca. 20 °C), das Abtrennen des Restlösungsmittels aus der Säurephase vorteilhafterweise durch sogenannte Flashverdampfung bei ca. 10 bis 30 °C und 2,66 bis 13,3 kPa (20 bis 100 Torr). Das durch Dekantation leicht und nahezu vollständig abgetrennte und durch Flash-Destillation rückgewonnene Lösungsmittel wird direkt wieder in der Sulfonierungsreaktion verwendet. Es entstehen somit praktisch keine Lösungsmittelverluste, was das Verfahren ausserordentlich ökologisch gestaltet.

Als reaktionsinerte, organische Lösungsmittel für das vorliegende Verfahren kommen vor allem Chlorkohlenwasserstoffe, wie z.B. Dichlormethan, Chloroform, Tetrachlormethan, 1,2-Dichloräthan und Chlorbenzol, in Betracht, wegen Energieersparnisüberlegungen aber insbesondere leicht siedende Chlorkohlenwasserstoffe. Bevorzugt ist Dichlormethan.

Das beschriebene Verfahren erweist sich als besonders vorteilhaft, indem sich die Hauptmenge des Lösungsmittels leicht abtrennen und direkt wiederverwenden lässt, das AS-AN in der Säurephase als pumpbarer Brei ohne Zwischenisolierung für die Weiterverarbeitung gut geeignet ist und das Produkt als Mg-Salz der Nitro-Armstrongsäure in hoher Reinheit und Gesamtausbeute erhalten wird.

Das für die Nitrierung bevorzugte simultane Dosieren des AS-AN-Breies und der gebräuchlichen Mischsäure zu einer Restmenge Nitriermasse ermöglich im technischen Masstab von Anbeginn an das Abführen der hohen Nitrierwärme und das Einhalten der geforderten Temperaturgrenzen von 10 bis 60 °C, vorzugsweise von 35 bis 45 °C. Anderenfalls wäre ein externer besonderer Kühlkreislauf erforderlich. Versuche haben nämlich ergeben, dass durch mechanische Einwirkung auf unreagiertes AS-AN dieses geschädigt wird, was sich in einer Ausbeuteminderung bemerkbar macht. Diese Arbeitsweise ist auch vorteilhaft, weil sich Dosierschwankungen nicht nachteilig auswirken und die Prozess-sicherheit selbst bei massiven Dosierfehlern aber auch bei anderen Störungen, wie z.B. Ausfall der Kühlung, stets gewährleistet ist.

Das Gesamtverfahren kann kontinuierlich, teilkontinuierlich oder chargenweise betrieben werden.

Das nachfolgende Beispiel veranschaulicht das neue Verfahren.

Beispiel

192,3 g Naphthalin (1,5 Mol) werden in 961,0 g Dichlormethan und gleichzeitig 360,3 g SO₃-flüssig in 1 540 g Dichlormethan gelöst. Die beiden Lösungen werden auf − 10 °C gekühlt und in vorgelegtes, ebenfalls auf − 10 °C gekühltes Dichlormethan (600 g) gleichzeitig so zulaufen gelassen, dass die beiden Mischungen in gleichem Zeitintervall zudosiert sind. Das Reaktionsgemisch wird durch Verdampfungskühlung bei 9,31 bis 10,64 kPa (70 bis 80 Torr) bei einer Temperatur zwischen − 5 und − 10 °C gehalten. Das Destillat aus der Verdampfungskühlung, total 1 209 g, wird zum Lösen von SO₃ wiederverwendet. Die Sulfonierungsreaktion ist nach ca. 1 bis 2 Stunden beendet. Man erhält eine Suspension des Sulfonierungsreaktionsgemisches.

In einem separaten Kolben werden nun 800 g rohes Nitrierungsreaktionsgemisch (Rückführung aus vorhergehendem Nitrieransatz) und 732 g $H_2SO_4$ 100% vorgelegt und vermischt. Zur Suspension aus der Sulfonierungsreaktion (1,17 Mol Naphthalin-1,5-disulfonsäure und 0,33 Mol isomerer Di- und Trisulfonsäure) wird nun bei 20 °C das Gemisch aus dem separaten Kolben zugegeben und langsam gerührt, bis sich das Dichlormethan in der oberen Schicht klar abtrennt, wonach es abgesaugt (dekantiert) werden kann. Das dadurch erhaltene Dichlormethan — ca. 1 400 g — wird ohne Aufarbeitung in die Sulfonierungsreaktion recycliert. Die restlichen 414 g Dichlormethan werden bei 10 bis 30 °C und 2,66 kPa (20 Torr) durch eine sogenannte Flash-Verdampfung abdestilliert und können ebenfalls wiederverwendet werden. Bei 2,66 kPa (20 Torr) wird die Temperatur des Destillationsrückstandes auf 20 °C gehalten und bei dieser Temperatur in die nachfolgende Nitrierung eingespeist.

Die Nitrierung erfolgt durch simultane Dosierung von 312 g Mischsäure und 2 083 g Destillationsrückstandsbrei bei 30 bis 40 °C in 2 400 g Restmenge des Nitriergemisches aus einem vorausgegangenen Ansatz. Danach lässt man unter ständigem Rühren bei 40 °C ausreagieren, was insgesamt ca. 4 Stunden in Anspruch nimmt.

Ein Teil = 800 g (ca. 1/6) der erhaltenen rohen Reaktionsmischung wird in die vorher beschriebene Zweiphasentrennung rückgeführt.

Ein zweiter Teil (ca. 2/6) wird folgendermassen zum Produkt aufgearbeitet : Im korrosionsfesten Reaktor mit Doppelmantel werden 1 123 g kaltes Wasser vorgelegt und innerhalb von 30 Minuten 1 594 g des Nitriergemisches unter starkem Rühren zugegeben. Durch leichtes Kühlen wird die Temperatur auf 100 °C gehalten. Das Reaktionsgemisch geht dabei vollständig in Lösung. Nach beendetem Zulauf erfolgt die selektive Fällung des Nitro-Armstrongsäure-Mg-Salzes durch Zudosieren von 654 g einer wässrigen Bittersalzlösung ($MgSO_4$-Gehalt 158,0 g) während 60 Minuten.

Danach werden während 5 Minuten 390 g Waschlauge aus einer vorhergehenden Fällung zugegeben. Die Suspension wird nun noch 60 Minuten bei 95 °C langsam gerührt, auf 60 °C abgekühlt und weitere 60 Minuten gerührt. Nun erfolgt die Filtration der grobkristallinen Suspension. Der Filterkuchen wird danach abgepresst und die Mutterlauge abgesaugt, wonach mit 300 g kaltem Wasser gewaschen und trockengesaugt wird. Mutter- und Waschlauge werden separat aufgefangen, da nur die Waschlauge recycliert wird.

Es werden ca. 590 g feuchtes 3-Nitronaphtha-lin-1,5-disulfonsäuredimagnesiumsalz erhalten, was 336,6 g Trockensubstanz entspricht (ca. 70 %, bezogen auf eingesetztes Naphthalin). Der Anteil an isomeren Nebenprodukten im Produkt ist kleiner als 1,5 %.

Ein dritter Teil, d.h. die Restmenge der rohe Nitrierungsreaktionsmischung (2 400 g, ca. 3/6) dient, insbesondere bei Durchführung des Verfahrens in technischem Masstab, wieder als Vorlage für einen weiteren nachfolgenden Nitrierungsansatz.

Wird bei der Nitrierungsreaktion die Mischsäure und der Destillationsrückstand nicht simultan sondern taktweise hintereinander so zugegeben, dass man jeweils kleine Anteile AS-AN-Brei vorlegt und die erforderliche Menge Mischsäure nachdosiert, so erhält man das Endprodukt in gleicher Ausbeute und Qualität.

**Ansprüche**

1. Verfahren zur Herstellung von 3-Nitro-naph-talin-1,5-disulfonsäure (Nitro-Armstrongsäure), dadurch gekennzeichnet, dass man Naphthalin in einem reaktionsinerten organischen Lösungsmittel mit flüssigen Schwefeltrioxid sulfoniert, dem erhaltenen Reaktionsgemisch eine Mischung, bestehend aus in etwa gleichen Teilen 100 %iger Schwefelsäure und ausreagierter roher Nitriermasse aus der Nitrierung einer vorausgegangenen Nitrierungscharge zusetzt, wodurch ein Zweiphasen-System resultiert, von dem die obere organischen Lösungsmittel-Phase dekantiert und der Rest des verbliebenen organischen Lösungsmittels aus dem Armstrongsäure anhydrid Säurebrei destillativ entfernt wird, wonach man zum Rückstand die zur Nitrierung erforderliche Salpetersäuremenge bei einer Temperatur zwischen 10 und 60 °C zusetzt und die entstandene 3-Nitronaphthalin-1,5-disulfonsäure durch Ausfällung und Filtration isoliert.

2. Ausführungsform des Verfahrens gemäss Anspruch 1, dadurch gekennzeichnet, dass man den nach der Phasentrennung erhaltenen Armstrongsäure anhydrid Brei und Salpetersäure simultan oder taktweise hintereinander in ein vorgelegtes rohes Nitriergemisch dosiert und anschliessend nitriert.

3. Ausführungsform des Verfahrens gemäss Anspruch 1, dadurch gekennzeichnet, dass man das rohe Nitriergemisch in 3 Teile aufteilt, wovon ein Teil zum Produkt 3-Nitronaphthalin-1,5-disulfonsäure aufgearbeitet wird, der zweite Teil mit 100 %iger Schwefelsäure im Verhältnis 1 : 1 gemisch wird und diesen zum Zwecke der Phasentrennung dem Sulfonierungsgemisch zugibt und der dritte Teil als Vorlage einer nächsten Nitrierungsreaktion verwendet.

4. Verfahren nach Ansprüchen 1 bis 3, dadurch gekennzeichnet, dass man das Lösungsmittel nach der Sulfierungsstufe vollständig entfernt.

5. Verfahren nach Ansprüchen 1 bis 3, dadurch gekennzeichnet, dass man die restlichen nach der Dekantation noch vorhandenen Lösungsmittelanteile erst während der Nitrierungsreaktionsstufe entfernt.

6. Verfahren nach Ansprüchen 1 bis 3, dadurch gekennzeichnet, dass das zur Phasentrennung nach der Sulfierungsreaktion einzusetzende Säuregemisch aus konzentrierter Schwefelsäure und ausreagierter roher Nitriermasse aus mindestens 6 Gewichtsteilen, und die beiden einzelnen Komponenten aus mindestens je 3 Gewichtsteilen pro 1 Teil in die Sulfonierungsreaktion eingesetztem Naphthalin bestehen.

7. Verfahren nach Ansprüchen 1 bis 6, dadurch gekennzeichnet, dass zur Nitrierung 1 bis 1,5 Mol Salpetersäure in Form von Mischsäure pro Mol Naphthalin verwendet wird.

8. Verfahren nach Ansprüchen 1 bis 7, dadurch gekennzeichnet, dass die zur Nitrierung verwendete Mischsäure 0,8 bis 1,2 Mol $SO_3$ pro Mol Salpetersäure enthält.

9. Verfahren gemäss Anspruch 1, dadurch gekennzeichnet, dass man die Phasentrennung Lösungsmittel-Säure kontinuierlich durchführt.

10. Verfahren gemäss Anspruch 1, dadurch gekennzeichnet, dass man die als Produkt entstandene 3-Nitronaphthalin-1,5-disulfonsäure nach vorherigem Lösen des Reaktionsgemisches in Wasser bei 90 bis 100 °C als Magnesiumsalz ausfällt.

**Claims**

1. A process for the production of 3-nitro-naphthalene-1,5-disulfonic acid (nitro-Armstrong's acid), which comprises sulfonating naphthalene in an inert organic solvent with liquid sulfur trioxide, adding to the reaction mixture a mixture consisting of about equal parts of 100 % sulfuric acid and fully reacted crude nitration mixture obtained from a previous nitration batch, resulting in the formation of a two-phase system from which the upper organic solvent phase is decanted and the remainder of the organic solvent is removed by distillation from the slurry of the Armstrong's acid anhydride, then adding to the residue the requisite amount of nitric acid for the nitration at a temperature between 10° and 60 °C, and isolating the resultant 3-nitronaphthalene-1,5-disulfonic acid by precipitation and filtration.

2. A modification of the process according to claim 1, which comprises adding the slurry of the Armstrong's acid anhydride obtained after the phase separation, and nitric acid, simultaneously or at intervals in succession, to a crude nitration mixture and subsequently nitrating the reaction mixture.

3. A modification of the process according to claim 1, which comprises dividing the crude nitration mixture into 3 portions, the first of said portions being worked up to give 3-nitro-naphthalene-1,5-disulfonic acid, the second being mixed with 100 % sulfuric acid in the ratio 1 : 1 and added to the sulfonation mixture for phase separation, and the third being employed for a subsequent nitration reaction.

4. A process according to claims 1 to 3, wherein the solvent is removed completely after the sulfonation stage.

5. A process according to claims 1 to 3, wherein the remaining solvent still present after decantation is removed during the nitration step.

6. A process according to claims 1 to 3, wherein the acid mixture of concentrated sulfuric acid and fully reacted crude nitration mixture required for the phase separation after the sulfonation reaction consists of at least 6 parts by weight, and each of the two individual components consists of at least 3 parts by weight, per 1 part of naphthalene employed in the sulfonation reaction.

7. A process according to claims 1 to 6, wherein 1 to 1.5 moles of nitric acid in the form of nitrosulfuric acid are used per mole of naphthalene for the nitration.

8. A process according to claims 1 to 7, wherein the nitrosulfuric acid used for the nitration contains 0.8 to 1.2 moles of $SO_3$ per mole of nitric acid.

9. A process according to claim 1, wherein the phase separation is carried out continuously.

10. A process according to claim 1, wherein the 3-nitronaphthalene-1,5-disulfonic acid obtained as product is precipitated in the form of the magnesium salt after dissolving the reaction mixture beforehand in water at 90° to 100 °C.

**Revendications**

1. Procédé pour la préparation de l'acide 3-nitro-naphtalène-1,5-disulfonique (acide nitro-Armstrong), caractérisé par le fait qu'on sulfone le naphtalène dans un solvant organique, inerte pour la réaction, avec du trioxyde de soufre liquide, qu'au mélange réactionnel obtenu on ajoute un mélange constitué en parties sensiblement égales d'acide sulfurique à 100 % et de masse nitrante brute ayant cessé de réagir provenant de la nitration d'une charge de nitration précédente, ce qui donne un système à deux phases à partir duquel on décante la phase solvant organique supérieur, et que le reste du solvant organique subsistant provenant de la suspension acide de l'anhydride d'acide Armstrong est éliminé par distillation, qu'on ajoute ensuite au résidu la quantité d'acide nitrique nécessaire pour la nitration, à une température comprise entre 10 et 60 °C, et qu'on isole l'acide-3-nitronaphtalène-1,5-disulfonique formé par précipitation et filtration.

2. Forme de réalisation du procédé selon la revendication 1, caractérisée par le fait qu'on dose la suspension de l'anhydride d'acide Armstrong, obtenue après la séparation en phases, et l'acide nitrique, simultanément ou successivement d'une façon rythmique, dans un mélange nitrant brut disposé au préalable puis qu'on effectue la nitration.

3. Forme de réalisation du procédé selon la revendication 1, caractérisée par le fait qu'on divise le mélange nitrant brut en trois parties, à partir desquelles une partie est transformée en le produit, à savoir l'acide 3-nitronaphtalène-1,5-disulfonique, la deuxième partie est mélangée avec l'acide sulfurique à 100 % dans le rapport 1 : 1 et ce mélange est ajouté au mélange de sulfonation en vue de la séparation en phases et la troisième partie est utilisée comme produit récepteur d'une réaction de nitration suivante.

4. Procédé selon les revendications 1 à 3, caractérisé par le fait qu'on élimine le solvant complètement après le stade de la sulfonation.

5. Procédé selon les revendications 1 à 3, caractérisé par le fait qu'on élimine la partie du solvant encore présente, restée après la décantation, seulement pendant le stade de la réaction de nitration.

6. Procédé selon les revendications 1 à 3, caractérisé par le fait que le mélange d'acides, ajouté en vue de la séparation en phases après la réaction de sulfonation, constitué par l'acide sulfurique concentré et la masse nitrante brute ayant cessé de réagir, constitue au moins 6 parties en poids, et que les deux composants pris séparément constituent chacun au moins 3 parties en poids, pour une partie de naphtalène mis en œuvre dans la réaction de sulfonation.

7. Procédé selon les revendications 1 à 6, caractérisé par le fait que pour la nitration, 1 à 1,5 mole d'acide nitrique est utilisée sous forme d'acide mélangé par mole de naphtalène.

8. Procédé selon les revendications 1 à 7, caractérisé par le fait que l'acide mélangé utilisé pour la nitration contient 0,8 à 1,2 mole de $SO_3$ par mole d'acide nitrique.

9. Procédé selon la revendication 1, caractérisé par le fait qu'on effectue la séparation en phases solvant-acide en continu.

10. Procédé selon la revendication 1, caractérisé par le fait qu'on précipite l'acide 3-nitronaphtalène-1,5-disulfonique, formé comme produit après dissolution préalable du mélange réactionnel dans l'eau à 90-100 °C, sous forme de sel de magnésium.